# FASCICULE DE BREVET EUROPEEN

(11) **EP 1 413 291 B1**
(45) Date de publication et mention de la délivrance du brevet: **03.05.2006**
(21) Numéro de dépôt: 03292514.1
(22) Date de dépôt: 10.10.2003
(51) Int. Cl.: A61K 8/63, A61Q 7/00, A61Q 19/00, A61Q 19/08

(54) **Utilisation de la 5-cholesten-3 béta,25-diol-7-one pour le traitement des peaux sèches ou du cuir chevelu sec**
Verwendung von 5-Cholesten-3 beta,25-diol-7-on zur Behandlung trockener Haut oder Kopfhaut
Use of 5-Cholesten-3 beta,25-diol-7-one for the treatment of dry skin or scalp

(30) Priorité: 24.10.2002 FR 0213339
(43) Date de publication de la demande: 28.04.2004
(73) Titulaire: L'OREAL, 75008 Paris (FR)
(72) Inventeur: Dalko, Maria, 91190 Gif S/Yvette (FR); Cavezza, Alexandre, 93290 Tremblay-en-France (FR)
(74) Mandataire: Kromer, Christophe

(56) Documents cités:
- EP-A- 0 608 600
- FR-A- 1 255 602
- US-A- 4 496 556
- TAYLOR, FREDERICK R. ET AL: "Correlation between oxysterol binding to a cytosolic binding protein and potency in the repression of hydroxymethylglutaryl coenzyme A reductase" JOURNAL OF BIOLOGICAL CHEMISTRY (1984), 259(20), 12382-7 , XP001179506
- DATABASE CA [en ligne] CHEMICAL ABSTRACTS SERVICE, COLUMBUS, OHIO, US; MUGIKURA, SHIGERU ET AL: "Cosmetics containing keratinization inhibitors" retrieved from STN Database accession no. 127:336482 XP002248377 -& JP 09 255552 A (SHISEIDO CO., LTD., JAPAN) 30 septembre 1997 (1997-09-30)
- DATABASE CA [en ligne] CHEMICAL ABSTRACTS SERVICE, COLUMBUS, OHIO, US; MARUYAMA, NAO ET AL: "Skin preparations containing keratin-softening agents and sequestering agents" retrieved from STN Database accession no. 131:276780 XP002248378 -& JP 11 279018 A (SHISEIDO CO., LTD., JAPAN) 12 octobre 1999 (1999-10-12)

## Description

La présente invention concerne un procédé cosmétique de traitement des peaux sèches ou du cuir chevelu sec, comprenant l'application topique sur la peau ou le cuir chevelu d'une composition renfermant, dans un milieu physiologiquement acceptable, au moins un oxystérol choisi parmi : la 5-cholesten-3β,25-diol-7-one et ses esters et éthers.

De nombreuses femmes à partir de trente-cinq ans, et plus particulièrement après la ménopause, se plaignent fréquemment du dessèchement de leur peau, et des manifestations d'inconfort ou inesthétiques qui en résultent (desquamation, teint terne, atonie cutanée). Or, ce dessèchement est dû, comme on le sait maintenant, à une diminution de la production de sébum avec l'âge.

Par ailleurs, les enfants dont la fonction sébacée n'est pas encore active présentent souvent des signes de peau sèche.

Le sébum est le produit naturel de la glande sébacée qui, conjointement à la sueur produite par les glandes eccrines ou aprocrines, constitue un hydratant naturel de l'épiderme. Il est constitué essentiellement d'un mélange plus ou moins complexe de lipides. Classiquement, la glande sébacée produit du squalène, des triglycérides, des cires aliphatiques, des cires de cholestérol et éventuellement du cholestérol libre (Stewart, M. E., Semin. Dermatol. 11, 100-105 (1992)). L'action des lipases bactériennes convertit une part variable des triglycérides en acides gras libres.

Le sébocyte constitue la cellule compétente de la glande sébacée. La production de sébum est associée au programme de différenciation terminale de cette cellule. Durant cette différenciation, l'activité métabolique du sébocyte est essentiellement axée sur la biosynthèse des lipides (la lipogénèse) et plus précisément sur la néosyntèse d'acides gras et du squalène.

Un composé permettant de stimuler la production des lipides constituant le sébum, par les cellules de la glande sébacée (les sébocytes), serait donc d'un intérêt certain pour le traitement des peaux sèches oligoséborrhéiques, c'est-à-dire présentant un taux de sébum inférieur à 100 µg/cm² au niveau du front.

A cette fin, il a été proposé dans le brevet US-4,496,556 d'utiliser la DHEA, un stéroïde sécrété par les glandes surrénales, ou ses esters, administrés par voie topique, pour augmenter la production de sébum.

Toutefois, pour des questions réglementaires, il n'est pas toujours possible d'utiliser ce type de composés dans le domaine cosmétique. En outre, son efficacité n'est pas suffisante sur les peaux oligoséborrhéiques. Il subsiste donc le besoin de disposer de composés cosmétiquement acceptables, permettant de stimuler efficacement la fonction sébacée en vue de traiter les peaux sèches oligoséborrhéiques.

De tels composés seraient également utiles dans le traitement des cuirs chevelus secs qui sont souvent associés à des cheveux ternes et atones.

La demanderesse a maintenant découvert avec étonnement que la 5-cholesten-3β,25-diol-7-one permettait de satisfaire ce besoin.

Il est, certes, connu d'utiliser certains dérivés de cholestérol à des fins cosmétiques, dans des compositons appliquées topiquement sur la peau. Ainsi, le document FR-1 255 602 divulgue des compositions destinées à prévenir la déshydratation des peaux sèches, en particulier dues à l'âge, comprenant comme adjuvant des stérols tels que le 7-oxo cholestérol. On connaît également du document EP-0 608 600 des composés destinés à remplacer les lipides cutanés, utilisables aussi dans le traitement des peaux sèches et des cuirs chevelus secs, dont le 7-oxo cholestérol.

Par ailleurs, il a été suggéré d'utiliser le 25-hydroxy cholestérol dans des compositions cosmétiques destinées notamment à conditionner la peau et à lutter contre les rides et la rugosité de la peau (JP-09 255 552 et JP-11 279018).

Toutefois, à la connaissance de la Demanderesse, il n'a encore jamais été suggéré d'application cosmétique d'oxystérols tels que la 5-cholesten-3β,25-diol-7-one ou des esters ou éthers de ce composé (identifiés ci-après par "dérivés").

La présente invention a donc pour objet un procédé cosmétique de traitement des peaux sèches ou du cuir chevelu sec, comprenant l'application topique sur la peau ou le cuir chevelu d'une composition renfermant, dans un milieu physiologiquement acceptable, au moins un oxystérol choisi parmi : la 5-cholesten-3β,25-diol-7-one et ses esters et éthers.

La 5-cholesten-3β,25-diol-7-one répond à la formule (I) suivante :

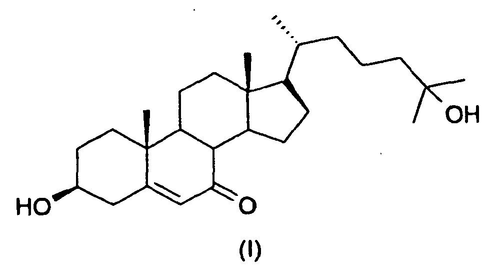

Ce composé est notamment disponible auprès de la société STERALOIDS sous la référence C6550.

La composition selon l'invention est particulièrement bien adaptée au traitement des peaux sèches oligoséborrhéiques, c'est-à-dire des peaux présentant un taux de sébum inférieur à 100 µg/cm² au niveau du front. Ce type de peau se rencontre fréquemment chez les femmes au voisinage de la ménopause, de sorte que la composition utilisée selon l'invention est de préférence appliquée sur des femmes de plus de quarante ans.

La présente invention a encore pour objet l'utilisation cosmétique d'au moins un oxystérol choisi parmi : la 5-cholesten-3β,25-diol-7-one et ses esters et éthers, en tant qu'agent pour le traitement des peaux sèches ou du cuir chevelu sec

Elle a également pour objet l'utilisation d'au moins un oxystérol choisi parmi : la 5-cholesten-3β,25-diol-7-one et ses esters et éthers, pour la préparation d'une composition, notamment dermatologique, destinée à traiter les désordres liés aux peaux sèches oligoséborrhéiques, en particulier les dermites.

La quantité d'oxystérol utilisable selon l'invention est bien entendu fonction de l'effet recherché et peut donc varier dans une large mesure. D'une manière générale, le ou les oxystérol(s) sera/seront présent(s) en une quantité suffisante pour augmenter significativement la production de sébum et avantageusement pour augmenter d'au moins 10% la production de sébum par une culture de sébocytes, comme décrit dans l'Exemple 1 ci-après.

Pour donner un ordre de grandeur, on peut utiliser ces oxystérols en une quantité représentant de 0,001% à 5% du poids total de la composition, préférentiellement en une quantité représentant de 0,05% à 1% du poids total de la composition.

La composition selon l'invention est généralement adaptée à une application topique sur la peau et/ou le cuir chevelu et elle contient donc un milieu physiologiquement acceptable, c'est-à-dire compatible avec la peau, ses phanères (cils, ongles, cheveux) et/ou les muqueuses.

Pour une application topique sur la peau, cette composition peut se présenter sous toutes les formes galéniques normalement utilisées dans les domaines cosmétique et dermatologique, et elle peut être notamment sous forme d'une solution huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau. Elle peut aussi être utilisée comme shampooing ou après-shampooing.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des oxystérols utilisés selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de camauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents apaisants ; et les agents stimulant la prolifération et/ou la différenciation des kératinocytes.

En effet, la stimulation de la séborrhée par les oxystérols et dérivés selon l'invention peut, chez certaines personnes, fournir un terrain de prolifération pour la microflore résidente de l'ostium folliculaire (Propionibacterium acnes en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut diverger vers une lésion acnéique inflammatoire.

L'ajout d'agents desquamants ou régulant la prolifération ou la différenciation des kératinocytes à la composition selon l'invention permettent d'éviter la formation de ces comédons. De même, des agents anti-bactériens ou bactériostatiques permettraient, en modérant la prolifération de la microflore résidente, d'obtenir le même effet.

En outre, les agents hydratants peuvent compléter l'effet obtenu à l'aide de l'oxystérol selon l'invention, et les agents apaisants sont utiles pour améliorer le confort des peaux sèches oligoséborrhéiques.

Des exemples de tels actifs additionnels sont donnés ci-dessous.

### Agents desguamants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des coméodesmosomes, les glycosidases, la stratum comeum chymotryptic enzym (SCCE) voire d'autres protéases (trypsine, chymotrypsine-like). On peut citer les agents chélatant des sels minéraux: l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanol-6-D-maltose et la N-acétyl glucosamine.

### Agent hydratant

Par "agent hydratant", on entend :
- soit un composé agissant sur la fonction barrière, en vue de maintenir l'hydratation du stratum comeum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine :

- soit un composé activant les glandes sébacées tel que la DHEA et ses dérivés, et la vitamine D et ses dérivés.

### Agents stimulant la prolifération et/ou la différenciation des kératinocytes

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les rétinoïdes sont préférés pour une utilisation dans cette invention, en particulier le rétinol et ses esters.

Les agents stimulant la différenciation des kératinocytes comprennent par exemple les minéraux tels que le calcium ; l'extrait de lupin commercialisé par la société SILAB sous la dénomination commerciale Photopréventine® ; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl® .

### Agents apaisants

Parmi les matières premières efficaces comme agents apaisants, on peut citer de façon non limitative les actifs suivants : les triterpènes pentacycliques, comme l'acide β-glycyrrhétinique et ses sels et/ou ses dérivés (acide glyyrrhetique monoglucuronide, stearyl glycyrrhetinate, acide 3- stéaroyloxy glycyrrhetique), l'acide ursolique et ses sels, l'acide oléanolique et ses sels, l'acide bétulinique et ses sels; les extraits de Paeonia suffruticosa et/ou lactiflora, de Rosmarinus officinalis, d'épilobe, de Pygeum, de Boswellia serrata, de Centipeda cunnighami, d'Helianthus annuus, de Cola nitida, de clou de girofle et de Bacopa moniera ; les sels de l'acide salicylique et en particulier le salicylate de zinc ; les extraits d'algues, en particulier de Laminaria saccharina ; l'huile de Canola, de Tamanu, de calophillum, les huiles insaturées en oméga 3 telles que les huiles de rosier muscat, de cassis, d'ecchium, de poisson ; l'α-bisabolol et les extraits de camomille ; l'allantoine ; le diesterphosphorique de vitamine E et C ; la capryloyl glycine ; les tocotrienols ; le piperonal ; l'aloe vera ; les phytostérols ; la cortisone, l'hydrocortisone, l'indometacine la bêta méthasone.

On peut citer en outre les antagonistes de substances P et notamment : les sels de strontium ; les eaux thermales et en particulier l'eau thermale du bassin de Vichy et l'eau thermale de La Roche Posay ; les extraits bactériens et en particulier l'extrait de bactéries filamenteuses non photosynthétiques décrit dans la demande de brevet EP-0 761 204, préparé de préférence à partir de bactéries appartenant à l'ordre des Beggiatoales, et plus particulièrement au genre Vitreoscilla. Préférentiellement, on utilise selon l'invention une souche de *Vitreoscilla filiformis.*

On peut également citer les antagonistes de CGRP et en particulier un extrait de cellules (de préférence indifférenciées) d'au moins un végétal de la famille des Iridacées, obtenu par culture *in vitro.* L'Iridacée appartient de préférence au genre Iris. En particulier, on préfère utiliser un extrait aqueux d'*Iris pallida,* comme décrit dans la demande EP-0 765 668.

On peut enfin citer les antagonistes de bradykinine et en particulier un extrait d'au moins un végétal de la famille des Rosacées, de préférence cultivé *in vivo.* Préférentiellement, on utilise selon l'invention un végétal appartenant au genre Rosa, avantageusement de l'espèce *Rosa gallica,* plus préférentiellement un extrait hydroglycolique de pétales de *Rosa gallica,* comme décrit dans la demande de brevet EP-0 909 556.

### Agents anti-bactériens

Les agents antibactériens susceptibles d'être utilisés dans la présente invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique, et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le famesol, les phytosphingosines et leurs mélanges.

Les agents antibactériens préférés sont le triclosan, le phénoxyéthanol, l'octoxyglycérine, l'octanoylglycine, l'acide 10-hydroxy-2-décanoïque; le caprylyl glycol, le famesol et l'acide azélaïque.

L'invention sera maintenant illustrée par les exemples non limitatifs suivants. Dans ces exemples, les quantités sont indiquées en pourcentage pondéral.

### EXEMPLES

### Exemple 1 : Mise en évidence de l'activité de la 5-cholesten-3β,25-diol-7-one sur la lipogénèse

On a testé la 5-cholesten-3β-25-diol-7-one, comparée à celle du 7-oxo cholestérol et du 125-hydroxy cholestérol, sur un modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020 (1999).

Le test a consisté à mesurer la quantité de lipides produite par les sébocytes de la lignée (à confluence), en présence ou non d'agent actif, dilué dans le DMSO, de telle sorte que la quantité finale de DMSO dans le milieu de culture soit 0,1%. Après 2 jours de traitement, les cellules adhérentes sont traitées par du Rouge de Nile (1 µg/ml). Le contenu en lipides est ensuite quantifié par mesure de la fluorescence du colorant (couple d'excitation/émission : 485-540nm pour les lipides neutres).

Les tests sont réalisés en sixplicates et l'expérience est renouvelée quatre fois.

En parallèle, des tests de prolifération (MUH) et de viabilité cellulaire (LDH) permettent de vérifier que les effets obtenus ne sont pas liés à une modification notable de ces paramètres biologiques.

Les résultats sont rassemblés dans le Tableau ci-dessous :

| CONCENTRATION EN COMPOSE TESTE | VARIATION DES LIPIDES (par rapport au témoin) |
|---|---|
| la 5-cholesten-3β,25-diol-7-one (10⁻⁴ M) | + 90% |
| la 5-cholesten-3β,25-diol-7-one (10⁻⁵ M) | + 85% |
| 7-oxo cholestérol (10⁻⁴ M) | + 20 % |
| 25-OH cholestérol (10⁻⁴ M) | 0 % |

Comme il ressort de ce Tableau, la 5-cholesten-3β,25-diol-7-one induit une augmentation importante de la lipogénèse sébocytaire, nettement supérieure à cette induite par d'autres dérivés de cholestérol, même à plus faible concentration que ces derniers.

En outre, dans le même test, la DHEA, testée à la concentration de 10⁻⁵ M n'a conduit qu'à une augmentation de 13 % du contenu en lipides du sébum. Les oxystérols selon l'invention sont donc plus efficaces que la DHEA.

### Exemple 2 : Composition cosmétique (émulsion H/E)

Cette composition est préparée de manière classique pour l'homme du métier. Les quantités données dans ces exemples sont indiquées en pourcentages pondéraux.

| | | |
|---|---|---|
| 5-cholesten-3β,25-diol-7-one | | 0.3 % |
| Extrait lyophilisé de romarin | | 0.2 % |
| Stéarate de glycérol | | 2 % |
| Polysorbate 60 | | 1 % |
| Acide stéarique | | 1.4 % |
| Triéthanolamine | | 0.7 % |
| Carbomer | | 0.4 % |
| Huile d'olive | | 12 % |
| Fraction liquide du beurre de karité | | 12 % |
| Octyldodécanol | | 6 % |
| Isononanoate d'isononyle | | 10 % |
| Antioxydant | | 0.05% |
| Parfum | | 0.5 % |
| Conservateur | | 0.3 % |
| Eau | qsp | 100 % |

Cette composition, utilisée en applications bi-quotidiennes, permet de redynamiser la fonction sébacée des peaux sèches.

## Revendications

1. Procédé cosmétique de traitement des peaux sèches ou du cuir chevelu sec, comprenant l'application topique sur la peau ou le cuir chevelu d'une composition renfermant, dans un milieu physiologiquement acceptable, au moins un oxystérol choisi parmi : la 5-cholesten-3β,25-diol-7-one et ses esters et éthers.

2. Procédé selon la revendication 1, **caractérisé en ce que** l'oxystérol est la 5-cholesten-3β,25-diol-7-one.

3. Procédé selon la revendication 1 ou 2, **caractérisé en ce que** l'oxystérol représente de 0,05% à 1% du poids total de la composition.

4. Procédé selon l'une quelconque des revendications 1 à 3, **caractérisé en ce que** lesdites peaux sèches sont des peaux sèches oligoséborrhéiques.

5. Procédé selon l'une quelconque des revendications 1 à 4, **caractérisé en ce que** ladite composition est appliquée sur des femmes de plus de quarante ans.

6. Utilisation cosmétique d'au moins un oxystérol choisi parmi : la 5-cholesten-3β,25-diol-7-one et ses esters et éthers, en tant qu'agent pour le traitement des peaux sèches ou du cuir chevelu sec.

7. Utilisation d'au moins un oxystérol choisi parmi : la 5-cholesten-3β,25-diol-7-one et ses esters et éthers, pour la préparation d'une composition destinée à traiter les désordres liés aux peaux sèches oligoséborrhéiques.

8. Utilisation selon la revendication 7, **caractérisée en ce que** lesdits désordres sont les dermites.

9. Composition comprenant, dans un milieu physiologiquement acceptable, au moins un oxystérol choisi parmi : la 5-cholesten-3β,25-diol-7-one et ses esters et éthers, et au moins un composé choisi parmi : un agent desquamant, un agent hydratant, un agent apaisant, un agent favorisant la prolifération et/ou la différenciation des kératinocytes et un agent anti-bactérien choisi parmi le triclosan, le phénoxyéthanol, l'octoxyglycérine, l'octanoylglycine, l'acide 10-hydroxy-2-décanoïque, le caprylyl glycol, le famesol et l'acide azélaïque.

10. Composition selon la revendication 9, **caractérisée en ce que** ledit agent desquamant est choisi parmi : l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ; l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que le méthyl glycine diacétate de sodium) ; le miel ; et les dérivés de sucre tels que l'O-octanoyl-6-D-maltose et la N-acétyl glucosamine.

11. Composition selon la revendication 9 ou 10, **caractérisée en ce que** ledit agent hydratant est choisi parmi : les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, la N-α-benzoyl-L-arginine ; la DHEA et ses dérivés ; et la vitamine D et ses dérivés.

12. Composition selon l'une quelconque des revendications 9 à 11, **caractérisée en ce que** ledit agent apaisant est choisi parmi : les triterpènes pentacycliques, comme l'acide β-glycyrrhétinique et ses sels et/ou ses dérivés, l'acide ursolique et ses sels, l'acide oléanolique et ses sels, l'acide bétulinique et ses sels ; les extraits de Paeonia suffruticosa et/ou lactiflora, de Rosmarinus officinalis, d'épilobe, de Pygeum, de Boswellia serrata, de Centipeda cunnighami, d'Helianthus annuus, de Cola nitida, de clou de girofle et de Bacopa moniera ; les sels de l'acide salicylique et en particulier le salicylate de zinc; les extraits d'algues, en particulier de Laminaria saccharina ; l'huile de Canola, de Tamanu, de calophillum, les huiles insaturées en oméga 3 telles que les huiles de rosier muscat, de cassis, d'ecchium, de poisson ; l'α-bisabolol et les extraits de camomille ; l'allantoine ; le diesterphosphorique de vitamine E et C ; la capryloyl glycine ; les tocotrienols ; le piperonal ; l'aloe vera ; les phytostérols ; la cortisone, l'hydrocortisone, l'indometacine la béta méthasone ; une souche de *Vitreoscilla filiformis,* un extrait aqueux d'*Iris pallida* ; et un extrait hydroglycolique de pétales de *Rosa gallica.*

13. Composition selon l'une quelconque des revendications 9 à 12, **caractérisée en ce que** ledit agent favorisant la prolifération des kératinocytes est un rétinoïde, tel que le rétinol et ses esters.

14. Composition selon l'une quelconque des revendications 9 à 13, **caractérisée en ce qu'**elle est sous la forme d'une émulsion huile-dans-eau.

## Claims

1. Cosmetic process for treating dry skin or a dry scalp, comprising the topical application to the skin or the scalp of a composition containing, in a physiologically acceptable medium, at least one oxysterol chosen from: 5-cholestene-3β,25-diol-7-one and esters and ethers thereof.

2. Process according to Claim 1, **characterized in that** the oxysterol is 5-cholestene-3β,25-diol-7-one.

3. Process according to Claim 1 or 2, **characterized in that** the oxysterol represents from 0.05% to 1% of the total weight of the composition.

4. Process according to any one of Claims 1 to 3, **characterized in that** the said dry skin is oligoseborrhoeic dry skin.

5. Process according to any one of Claims 1 to 4, **characterized in that** the said composition is applied to women over forty years old.

6. Cosmetic use of at least one oxysterol chosen from: 5-cholestene-3β,25-diol-7-one and esters and ethers thereof, as an agent for treating dry skin or a dry scalp.

7. Use of at least one oxysterol chosen from: 5-cholestene-3β,25-diol-7-one and esters and ethers thereof, for the preparation of a composition for treating disorders associated with oligoseborrhoeic dry skin.

8. Use according to Claim 7, **characterized in that** the said disorders are dermatitides.

9. Composition comprising, in a physiologically acceptable medium, at least one oxysterol chosen from: 5-cholestene-3β,25-diol-7-one and esters and ethers thereof, and at least one compound chosen from: a desquamating agent, a moisturizer, a calmative, an agent for promoting keratinocyte proliferation and/or differentiation, and an antibacterial agent chosen from triclosan, phenoxyethanol, octoxyglycerol, octanoylglycine, 10-hydroxy-2-decanoic acid, caprylyl glycol, farnesol and azeleic acid.

10. Composition according to Claim 9, **characterized in that** the said desquamating agent is chosen from: salicylic acid and its derivatives (including 5-n-octanoylsalicylic acid); α-hydroxy acids, such as glycolic acid, citric acid, lactic acid, tartaric acid, malic acid or mandelic acid; urea; gentisic acid; oligofucoses; cinnamic acid; extract of *Saphora japonica*; resveratrol; EDTA; N-acyl-N,N'N'-ethylenediaminetriacetic acid; aminosulfonic compounds and in particular (N-2-hydroxyethylpiperazine-N-2-ethane)-sulfonic acid (HEPES); 2-oxothiazolidine-4-carboxylic acid (procysteine) derivatives; α-amino acid derivatives of glycine type (such as sodium methyl glycine diacetate); honey; and sugar derivatives such as O-octanoyl-6-D-maltose and N-acetylglucosamine.

11. Composition according to Claim 9 or 10, **characterized in that** the said moisturizer is chosen from: ceramides, sphingoid-based compounds, lecithins, glycosphingolipids, phospholipids, cholesterol and its derivatives, phytosterols (stigmasterol, β-sitosterol, campesterol), essential fatty acids, 1,2-diacylglycerol, 4-chromanone, pentacyclic triterpenes such as ursolic acid, petroleum jelly and lanolin; threalose and its derivatives, hyaluronic acid and its derivatives, glycerol, pentanediol, sodium pidolate, serine, xylitol, sodium lactate, polyglyceryl acrylate, ectoin and its derivatives, chitosan, oligosaccharides and polysaccharides, cyclic carbonates, N-lauroylpyrrolidonecarboxylic acid, N-α-benzoyl-L-arginine; DHEA and its derivatives; and vitamin D and its derivatives.

12. Composition according to any one of Claims 9 to 11, **characterized in that** the said calmative is chosen from: pentacyclic triterpenes, for instance β-glycyrrhetinic acid and salts and/or derivatives thereof, ursolic acid and its salts, oleanolic acid and its salts, betulinic acid and its salts; extracts of Paeonia *suffruticosa* and/or *lactiflora,* Rosmarinus *officinalis,* willowherb, *Pygeum, Boswellia serrata, Centipeda cunninghami, Helianthus annuus, Cola nitida,* clove and *Bacopa moniera;* salicylic acid salts and in particular zinc salicylate; algal extracts, in particular of *Laminaria saccharina;* canola oil, tamanu oil or beauty-leaf oil, omega-3 unsaturated oils such as musk rose oil, blackcurrant oil, ecchium oil or fish oil; α-bisabolol and camomile extracts; allantoin; the phosphoric diester of vitamins E and C; capryloyl glycine; tocotrienols; piperonal; Aloe vera; phytosterols; cortisone, hydrocortisone, indomethacin and betamethasone; a strain of *Vitreoscilla filiformis,* an aqueous extract of *Iris pallida;* and a water-glycol extract of *Rosa gallica* petals.

13. Composition according to any one of Claims 9 to 12, **characterized in that** the said agent for promoting keratinocyte proliferation is a retinoid, such as retinol and its esters.

14. Composition according to any one of Claims 9 to 13, **characterized in that** it is in the form of an oil-in-water emulsion.

## Patentansprüche

1. Kosmetisches Verfahren zur Behandlung von trockener Haut oder trockener Kopfhaut, das die topische Anwendung einer Zusammensetzung auf der Haut oder der Kopfhaut umfasst, wobei die Zusammensetzung in einem physiologisch akzeptablen Medium mindestens ein Oxysterin enthält, das unter 5-Cholesten-3β,25-diol-7-on und seinen Estern und Ethern ausgewählt ist.

2. Verfahren nach Anspruch 1, **dadurch gekennzeichnet, dass** das Oxysterin 5-Cholesten-3β,25-diol-7-on ist.

3. Verfahren nach Anspruch 1 oder 2, **dadurch gekennzeichnet, dass** das Oxysterin 0,05 bis 1 % des Gesamtgewichts der Zusammensetzung ausmacht.

4. Verfahren nach einem der Ansprüche 1 bis 3, **dadurch gekennzeichnet, dass** die trockene Haut trockene oligoseborrhoische Haut ist.

5. Verfahren nach einem der Ansprüche 1 bis 4, **dadurch gekennzeichnet, dass** die Zusammensetzung bei Frauen von über vierzig Jahren angewandt wird.

6. Kosmetische Verwendung mindestens eines Oxysterins, das unter 5-Cholesten-3β, 25-diol-7-on und seinen Estern und Ethern ausgewählt ist, als Mittel zur Behandlung von trockener Haut und trockener Kopfhaut.

7. Verwendung mindestens eines Oxysterins, das unter 5-Cholesten-3β,25-diol-7-on und seinen Estern und Ethern ausgewählt ist, zur Herstellung einer Zusammensetzung zur Behandlung von Störungen, die mit trockener oligoseborrhoischer Haut verbunden sind.

8. Verwendung nach Anspruch 7, **dadurch gekennzeichnet, dass** die Störungen Dermitiden sind.

9. Zusammensetzung, die in einem physiologisch akzeptablen Medium enthält: mindestens ein Oxysterin, das unter 5-Cholesten-3β-25-diol-7-on und seinen Estern und Ethern ausgewählt ist, und mindestens eine Verbindung, die ausgewählt ist unter: einem Desquamationsmittel, einem Hydratationsmittel, einem reizlindernden Mittel, einem Mittel, das die Proliferation und/oder die Differenzierung von Keratinocyten begünstigt, sowie ein antibakterielles Mittel, das ausgewählt ist unter: Triclosan, Phenoxyethanol, Octoxyglycerin, Octanoylglycin, 10-Hydroxy-2-decansäure, Caprylylglykol, Farnesol und Azelainsäure.

10. Zusammensetzung nach Anspruch 9, **dadurch gekennzeichnet, dass** das Desquamationsmittel ausgewählt ist unter: Salicylsäure und ihren Derivaten (wozu n-Octanoyl-5-salicylsäure gehört); α-Hydroxysäuren, wie Glykolsäure, Citronensäure, Milchsäure, Weinsäure, Äpfelsäure oder Mandelsäure; Harnstoff, Gentisinsäure; Oligofucosen, Zimtsäure, Extrakt von Saphorajaponica; Resveratrol; EDTA; N-Acyl-N,N',N'-ethylendiamintriessigsäure; Aminosulfonsäureverbindungen und insbesondere N-2-Hydroxyethylpiperazin-N-2-ethan-sulfonsäure (HEPES); Derivaten von 2-Oxothiazolidin-4-carbonsäure (Procystein); Derivaten von α-Aminosäuren vom Glycintyp (wie Natrium-Methylglycindiacetat); Honig; Zuckerderivaten wie O-Octanoyl-6-D-maltose und N-Acetylglucosamin.

11. Zusammensetzung nach Anspruch 9 oder 10, **dadurch gekennzeichnet, dass** das Hydratationsmittel ausgewählt ist unter: Ceramiden, Verbindungen auf Sphingoidbasis, Lecithinen, Glycosphingolipiden, Phopholipiden, Cholesterin und seinen Derivaten, Phytosterinen (Stigmasterin, β-Sitosterin, Campesterin), essentiellen Fettsäuren, 1,2-Diacylglycerin, 4-Chromanon, pentacyclischen Triterpenen wie Ursolsäure, Vaseline und Lanolin; Trehalose und ihren Derivaten, Hyaluronsäure und ihren Derivaten, Glycerin, Pentandiol, Natriumpidolat, Serin, Xylit, Natriumlactat, Glycerinpolyacrylat, Ectoin und seinen Derivaten, Chitosan, Oligo- und Polysacchariden, cyclischen Carbonaten, N-Lauroylpyrrolidoncarbonsäure, N-α-Benzoyl-L-arginin; DHEA und ihren Derivaten; Vitamin D und seinen Derivaten.

12. Zusammensetzung nach einem der Ansprüche 9 bis 11, **dadurch gekennzeichnet, dass** das reizlindernde Mittel ausgewählt ist unter: pentacyclischen Triterpenen, wie β-Glycyrrhetinsäure und ihren Salzen und/oder ihren Derivaten, Ursolsäure und ihren Salzen, Oleanolsäure und ihren Salzen, Betulinsäure und ihren Salzen; Extrakten von Paeonia suffruticosa und/oder lactiflora, Rosmarinus officinalis, Epilobium, Pygeum, Boswellia serrata, Centipeda cunnighami, Helianthus annuus, Cola nitida, Gewürznelken und Bacopa moniera; Salzen von Salicylsäure und insbesondere Zinksalicylat; Algenextrakten, insbesondere von Laminaria saccharina; Canolaöl, Tamanuöl, Calophyllumöl, ω-3-ungesättigten Ölen, wie Muskatrosenöl, Cassisöl, Echiumöl, Fischöl; α-Bisabolen und Kamillenextrakten; Allantoin; dem Phosphorsäurediester von Vitamin E und C; Capryloylglycin; Tocotrienolen; Piperonal; Aloe vera; Phytosterinen; Cortison, Hydrocortison; Indometacin, Betamethason; einem Stamm von Vitreoscilla filiformis, einem wässerigen Extrakt von Iris pallida; einem wässerig-glykolischen Extrakt von Blütenblättern von Rosa gallica.

13. Zusammensetzung nach einem der Ansprüche 9 bis 12, **dadurch gekennzeichnet, dass** das Mittel, das die Proliferation von Keratinocyten begünstigt, ein Retinoid ist, wie z.B. Retinol und seine Ester.

14. Zusammensetzung nach einem der Ansprüche 9 bis 13, **dadurch gekennzeichnet, dass** sie in Form einer Öl-in-Wasser-Emulsion vorliegt.
